(19) Europäisches Patentamt
European Patent Office
Office européen des brevets

(11) EP 0 600 793 B1

(12) **EUROPEAN PATENT SPECIFICATION**

(45) Date of publication and mention
of the grant of the patent:
**12.03.1997 Bulletin 1997/11**

(51) Int. Cl.$^6$: **B01D 71/80**, B01D 71/56,
B01D 69/08, C08G 69/40,
A61L 33/00, A61M 1/16

(21) Application number: **93402912.5**

(22) Date of filing: **01.12.1993**

(54) **Blood compatible materials**

Blutverträgliche Materialien

Matériaux hémocompatibles

(84) Designated Contracting States:
**BE DE FR GB IT NL SE**

(30) Priority: **01.12.1992 JP 321968/92**
**31.03.1993 JP 74083/93**

(43) Date of publication of application:
**08.06.1994 Bulletin 1994/23**

(73) Proprietor: **TERUMO KABUSHIKI KAISHA**
**Tokyo (JP)**

(72) Inventors:
• **Mochizuki, Akira**
**Hadano-shi, Kanagawa (JP)**
• **Nakazaki, Tomomichi**
**Fujisawa-shi, Kanagawa (JP)**
• **Saiga, Nobuko**
**Hadano-shi, Kanagawa (JP)**

• **Seita, Yukio**
**Hiratsuka-shi, Kanagawa (JP)**

(74) Representative: **Gillard, Marie-Louise et al**
**Cabinet Beau de Loménie**
**158, rue de l'Université**
**75340 Paris Cédex 07 (FR)**

(56) References cited:
**EP-A- 0 006 030**     **EP-A- 0 465 306**
**US-A- 4 873 292**     **US-A- 4 963 165**

• **DATABASE WPI Week 9313, Derwent**
**Publications Ltd., London, GB; AN 93-104364 &**
**JP-A-5 043 684 (MITSUBISHI KASEI CORP) 23**
**February 1993**
• **DATABASE WPI Week 8320, Derwent**
**Publications Ltd., London, GB; AN 76-65528X &**
**JP-B-58 020 647 (UNITIKA KK) 25 April 1983**

**Description**

BACKGROUND OF THE INVENTION

This invention relates to novel blood compatible materials that are made of thermoplastic polyether-polyamide block copolymers or polymer alloys containing said block copolymers. The invention also relates to hollow-fiber membranes formed of these blood compatible materials, as well as a process for producing such hollow-fiber membranes.

Quite a large number of investigations have already been conducted on blood compatible materials and active R&D efforts are still in progress in many fields of application. Blood compatible materials as adapted for use in medical devices can be utilized at various sites of contact with blood, such as artificial kidneys, oxygenator , catheters, blood filters, blood circuits and blood bags, but the device in which blood compatible materials find the greatest potential for use and far-reaching effects would be artificial kidneys.

Cellulose is one of the major materials for hemodialysis membranes that are currently employed in artificial kidneys and synthetic polymers used for the same purpose include polyacrylonitrile, polymethyl methacrylate, polysulfones, polyamides and ethylene-vinyl alcohol copolymers. Regenerated cellulose membranes as produced by precipitation of cellulose from a cuprammonium solution are used most extensively not only because they exhibit ultrafiltration rates that are preferred for hemodialysis and appropriate permeabilities to low-molecular weight solutes but also because they have a long history of use. However, regenerated cellulose membranes have been known to have various disadvantages, such as insufficient ability to reject from blood the deleterious substances of low to medium molecular weights as typified by $\beta_2$-microglobulin, the decrease in platelet counts and the transient decrease in leukocyte counts as observed during dialysis, and the activation of the complement system which is caused by hydroxyl groups in cellulose. Membranes that are composed of the synthetic polymers listed above are already on the commercial market; however, for the reason that they are essentially made of engineering materials as merely adapted for medical purposes, those synthetic-polymer membranes are either poor in biocompatibility or insufficient in mechanical strength or poor in permeability.

As a result of the active efforts that have recently been made to study blood compatible materials, it has come to be known that block copolymers having a microphase separating structure have excellent blood compatibility ("One Point Series of New Polymeric Materials", Vol. 11, "Biocompatible Polymers", 1988, Kyoritsu Shuppan).

Examples of the review on the use of polyether containing block copolymers in permselective membranes are US-A-4,069,151 and US-A-4,075,108, which teach membranes that are made from polyether-polycarbonate block copolymers. Examined Japanese Patent Publication (kokoku) No. Sho 63-25802 teaches a permselective membrane that is made from a caprolactam-polyalkylene ether-polyacyllactam block terpolymer. These permselective membranes are, however, short of perfection in operational aspects such as membrane performance and blood compatibility. The use of polyether-polyamide block copolymers in permselective membranes is proposed by Examined Japanese Patent Publication (kokoku) No. Sho 58-20647 but the only specific example that is given there is a polyethylene oxide-nylon 6 block copolymer and the permeability of the membrane that is made from this copolymer is quite unsatisfactory. EP-A-405306 shows a polyalkylene oxide-nylon 610 block copolymer as an example of the material of permselective membrane; although the membrane made of this block copolymer is shown to have good blood compatibility, its permeability is still unsatisfactory.

SUMMARY OF THE INVENTION

The present invention has been accomplished under these circumstances and has as an object providing a novel blood compatible material comprising a thermoplastic polyether-polyamide block copolymer.

Another object of the present invention is to provide a permselective membrane, particularly, a medical blood purifying membrane, that is derived from that novel blood compatible material.

The present inventors made various studies in order to locate the reason why satisfactory performance could not be exhibited by membranes that used conventional blood compatible materials which were made of polyether-polyamide block copolymers as based on aliphatic polyamides. As a result, the inventors reached the conclusion that the primary reason for the failure was the too strong force of cohesion between molecules. Stated more specifically, polyamides form strong intermolecular hydrogen bonds due to amide groups (they have strong intermolecular cohesion) and, hence, the molecules will arrange regularly by themselves to readily form a crystalline phase. This characteristic was assumed to be responsible for the difficulty involved in controlling the ultrafine structure of the separation active layer when manufacturing a membrane. Based on these observations, the present inventors noted the heat of crystallization, $\Delta Hc$, of polyamides as a measure for estimating the force of cohesion between their molecules and continued the studies, which eventually led to the accomplishment of the present invention.

Thus, in accordance with its first aspect, the present invention provides a blood compatible material that comprises a polyether-polyamide block copolymer comprising polyether blocks and polyamide blocks having a heat of crystallization of no more than 30 mJ/mg, wherein the polyether-polyamide block copolymer has thermal plasticity.

In accordance with its second aspect, the present invention provides a blood compatible material that comprises a polymer alloy that comprises a polyether-polyamide block copolymer which comprises polyether blocks and polyamide blocks having a heat of crystallization of no more than 30 mJ/mg, and, a polyamide having a heat of crystallization of no more than 30 mJ/mg.

In accordance with its third aspect, the present invention provides a permselective membrane that is made of the above-mentioned blood compatible material.

In accordance with its fourth aspect, the present invention provides a hollow-fiber membrane that is made of the above-mentioned blood compatible material.

In a preferred embodiment, said hollow-fiber membrane comprises at least an active layer which is located on inner surface or outer surface of the hollow-fiber, a finger like void and porous portion which is located between the active layer and the opposite side of the active layer.

In a preferred embodiment, said hollow-fiber membrane comprises at least an active layer which is located on inner surface or outer surface of the hollow-fiber, and a porous portion that is located inner or outer side to said active layer and which has a microporous structure with continuous open pores.

In accordance with its fourth aspect, the present invention provides a process for producing a hollow-fiber membrane that comprises the steps of preparing a spinning solution that consists of 5 - 40 wt% of the above-mentioned block copolymer or polymer alloy and 95 - 60 wt% of a polar solvent and extruding the spinning solution through the outer tube of a spinneret while, at the same time, extruding a coagulating solution through the inner tube of said spinneret, thereby coagulating the spinning solution.

In a preferred embodiment, said spinning solution may further contain a water-soluble polymer in an amount of 2 - 20 parts by weight per 98-80 parts by weight of said spinning solution.

In accordance with its fifth aspect, the present invention provides a hollow-fiber membrane that is produced by the above-described process or its preferred embodiment and which comprises at least an interior active layer and a porous portion that is located exterior to said active layer and which has a microporous structure with continuous open pores that extends from the interior towards the outermost surface.

BRIEF DESCRIPTION OF THE DRAWINGS

Fig. 1 is a graph showing the SC curve for the aqueous solution of dextran that was treated by the hollow-fiber membrane as prepared in Example 42;
Fig. 2 is a graph showing the SC curve for the aqueous solution of dextran that was treated by the hollow-fiber membrane as prepared in Example 52;
Fig. 3 is a scanning electron micrograph of a cross section of the hollow fiber that was prepared in Example 42;
Fig. 4 is a scanning electron micrograph of a cross section of the hollow fiber that was prepared in Example 52;
Fig. 5 is a perspective view showing the setup of an apparatus for measuring the water flow rate (UFR) of a flat-sheet membrane, in which numerals 1 and 1' each denote a cell for evaluating the water permeation of the membrane, 2 is an O-ring, 3 is the flat-sheet membrane, and 4 is a mesh screen;
Fig. 6 is a diagram showing schematically the circuit for measuring the water flow rate of a hollow fiber; and
Fig. 7 is a diagram showing schematically the circuit for measuring the sieve coefficients for albumin and an aqueous solution of dextran, in which numeral 1 denotes a thermostatic bath, 2 is a tubular membrane, 3 is physiological saline, 4 is a rotary pump, 5a and 5b are each a pressure gauge, 6 is a minimodule, 7 and 8 are each a tube 9 is a filtrate receptacle, and 10 is a waste sump.

DETAILED DESCRIPTION OF THE INVENTION

The present invention is described below in detail.

In its first aspect, the present invention relates to a blood compatible material that comprises thermoplastic polyether-polyamide block copolymer or a polymer alloy that consists of said polyether-polyamide block copolymer and a polyamide.

The polyamide which is one of the essential constituents of the present invention is synthesized by polycondensation between a dicarboxylic acid and a diamine and/or lactam ($\omega$-aminocarboxylic acid) and it is critical that this polyamide have a heat of crystallization, $\Delta Hc$, of no more than 30, preferably 0-28 mJ/mg. Aliphatic polyamides such as nylon 6, nylon 66, nylon 610 and nylon 12 have $\Delta Hc$ values of about 40 - 70 mJ/mg and the force of cohesion between their molecules is so strong that they exhibit an extremely high degree of crystallinity. The value of "heat of crystallization" can be determined with a differential scanning calorimeter (DSC) on the basis of the area of an exothermic peak that first appears when a molten polyamide is cooled at a rate of 5°C/min. While there are several ways to reduce the heat of crystallization, three common methods are the copolymerization of two or more diamines and/or dicarboxylic acids, lowering the density of amide groups, and introducing a substituent for nitrogen into amide groups. The copolymerization technique is the simplest but if it is practiced on aliphatic monomers, the product has such a low mechanical strength that it is not suitable for use as a medical material. In order to reduce the heat of crystallization while retaining sufficient mechanical properties to warrant the use of the polymer as a medical material, introducing recurring units having a cyclic structure is particularly effective. This would be because the cyclic structure introduced into the polymer will make it rigid and further interfere with the hydrogen bonds between amide groups due to steric hindrance.

If the blood compatible material of the present invention is to be used as a material for permselective membrane, it must have a heat of crystallization, ΔHc, of no more than 30 mJ/mg, preferably between 0 and 28 mJ/mg, in order for the desired membrane performance (permeability to solutes) to be manifested. The probable reason why the membrane performance is improved when the heat of crystallization is held to no more than 30 mJ/mg is that the force of cohesion between molecules is reasonably suppressed to insure that the controlled ultrafine structure of the separation active layer which is formed when the membrane is produced by immersion in a coagulation phase is kept in a stable state. On the other hand, the aliphatic polyamide containing block copolymers that are taught in the known references, supra have such a strong force of cohesion between molecules that it is difficult to control the ultrafine structure in the process of membrane production but, instead, the product membrane becomes too dense to exhibit good performance.

The major characteristic feature of the present invention is to use polyamides having a heat of crystallization, ΔHc, between 0 and 30 mJ/mg and such polyamides correspond to amorphous polyamides or crystalline polyamides having low to medium degrees of crystallinity.

Since the blood compatible material of the present invention is intended to be used in the medical field after sterilization, it desirably has the ability to withstand sterilization by autoclaving (at 121°C). Therefore, if the polyamides to be used in the present invention are amorphous, they have desirably a glass transition point of at least 160°C but this is not the case if sterilization is accomplished with EOG or γ-rays. It should be noted here that the polyamide is not necessarily amorphous even if its ΔHc is zero. This is because ΔHc is determined by the balance between the rate of crystallization and the rate at which the temperature is lowered on DSC. Whether the polyamide is crystalline or amorphous is desirably verified by checking as to whether it produces a peak for the fusion (or melting) of crystal. An alternative method of verification is by X-ray diffraction. The heat of melting of crystal, ΔHm, is not preferred as an absolute measure since it varies greatly with the thermal history of the sample. However, if annealed adequately, the polyamides that can be used in the present invention exhibit ΔHm values of approximately 40 mJ/mg and less and the range of about 20 - 40 mJ/mg is particularly desirable for effecting sterilization by autoclaving.

The polyamides have preferably an arocyclic or alicyclic structure. Recurring units having a cyclic structure can be introduced into the polyamide chain by various methods, such as (1) combining a diamine having a cyclic structure with an aliphatic dicarboxylic acid, (2) combining an aliphatic diamine with a dicarboxylic acid having a cyclic structure, (3) combining a diamine having a cyclic structure with a dicarboxylic acid also having a cyclic structure, and (4) using an aminocarboxylic acid having a cyclic structure. It should be noted that for the purpose of suppressing the force of cohesion between molecules, the recurring units having a cyclic structure desirably account for 100 - 30 mol%, of all recurring units in the polyamide block under consideration.

Specific examples of the diamine component having a cyclic structure include metaxylylenediamine, paraxylylenediamine, diaminocyclohexane, 4,4'-diaminodicyclohexylenemethane, 4,4'-diamino-3,3'-dimethyldicyclohexylenemethane, 4,4'-diaminodicyclohexylenepropane, isophoronediamine, 4,4'-diaminodiphenylmethane, tolylenediamine, phenylenediamine, diaminodiphenylether, 1,3-bisaminomethylcyclohexane and 1,4-bisdiaminomethylcyclohexane It is optional to use more than one kind of diamines having a cyclic structure when synthesizing the polyamides to be used in the present invention. Among the diamines listed above, wholly aromatic amines such as tolylenediamine and phenylenediamine which have no aliphatic side chains are condensed with isophthalic acid or terephthalic acid, the resulted polyamide are difficult to shape in a molten state; on the other hand, metaxylylenediamine and bisaminomethylcyclohexane are desired for various reasons such as easy availability, low price and high reactivity.

Specific examples of the dicarboxylic acid component having a cyclic structure include cyclohexanedicarboxylic acid, isophthalic acid, terephthalic acid, biphenylcarboxylic acid, 4,4'-dicarboxydiphenylether, naphthalenedicarboxylic acid, phenylenedipropionic acid and (p-carboxymethoxy)phenylpropionic acid. It is optional to use more than one kind of dicarboxylic acids having a cyclic structure but isophthalic acid and terephthalic acid are desirable from the viewpoints of price and purity.

A specific example of the acid and amine combination that produces the recurring units having a cyclic structure which are one of the characteristic features of the polyamide blocks used in the present invention is to combine at least one diamine having a cyclic structure as selected from the group of the diamines listed above with an aliphatic dicarboxylic acid having 4 or more carbon atoms. Specific examples of the dicarboxylic acid having 4 or more carbon atoms include adipic acid, suberic acid, azelaic acid, sebacic acid and dodecanoic diacid. Among these acids, adipic acid, azelaic acid and sebacic acid are preferred from various viewpoints such as control on the density of amide groups in the polyamide blocks and price. Other examples of the acid and amine combination that can be adopted include: the combination of at least one dicarboxylic acid as selected from the group of the above-listed dicarboxylic acids having a cyclic structure with an aliphatic diamine such as hexamethylenediamine, trimethylhexamethylenediamine or bis(3-aminopropyl)ether; and the combination of at least one each of dicarboxylic acid and diamine as selected from the groups of the above-listed dicarboxylic acids and diamines which have a cyclic structure. The recurring units having a cyclic structure can also be prepared by using aminocarboxylic acids (lactams) having a cyclic structure such as 4-aminomethylcyclohexanecarboxylic acid and 4-aminomethylbenzoic acid. These four methods may optionally be combined in any suitable way.

In the present invention, those recurring units which do not have a cyclic structure account for 0 - 70 mol% of all recurring units in the polyamide blocks. Such recurring units having no cyclic structure are produced from the combinations of aliphatic dicarboxylic acids such as adipic acid and sebacic acid that include ordinary dimer acids with aliphatic diamines, and/or from aliphatic lactams (ω-aminocarboxylic acids) such as caprolactam and laurolactam.

The polymer alloy to be used in the present invention which consists of the polyether-polyamide block copolymer that has been described above and the polyamide that has a heat of crystallization of no more than 30 mJ/mg is produced by blending said polyether-polyamide block copolymer with said polyamide. While the amount of the polyamide to be blended is not limited to any particular value, the said value may be such that the content of the polyether blocks be 0.5-60 wt% of the polymer alloy.

The polyamide to be blended may be the same or different from the polyamide which constitutes the polyamide blocks in the polyether-polyamide block copolymer. In a specific example, the block copolymer is polytetramethylene glycol-bisaminomethylcyclohexane-isophthalic acid (PTMG-NyBI) and this is to be alloyed with a polyamide that may be bisaminomethylcyclohexane-isophthalic acid (NyBI), bisaminomethylcyclohexane-isophthalic acid/hexamethylenediamine-isophthalic acid (NyBI/6I), or hexamethylenediamine-isophthalic acid/hexamethylenediamine-terephthalic acid (Ny6I/6T). The symbols used in the designations of the respective compounds have the following meanings: PTMG, polytetramethylene glycol; B, bisaminomethylcyclohexane; I, isophthalic acid; T, terephthalic acid; and 6, hexamethylenediamine.

Examples of the polyether chain to be used in the block copolymer under discussion include polyethylene glycol, polytetramethylene glycol, polypropylene glycol, propylene glycol - ethylene glycol copolymer and tetramethylene glycol-ethylene glycol copolymer. The present invention requires that these polyether chains be bound to the polyamide chains by covalent bonds and to meet this need, condensation reaction is desirably performed from such viewpoints as synthesis method. The polyether chains that can be used are those which terminate with an amino, carboxyl, hydroxyl or an isocyanato group. The terminal of the polyamide that reacts with the reactive terminal of said polyether is an amino or carboxyl group that originate from the starting material and, therefore, in terms of stability and other aspects, it is desirable for the reactive group in said polyether to be an amino or carboxyl group and that amido bonds are generated to have the polyether chain bind with the polyamide chain. If the polyether terminates with a hydroxyl group, the mode of binding is by ester bonds and, hence, problems may sometimes arise in such aspects as heat stability and resistance to hydrolysis. If the polyether terminates with an isocyanato group, it is so reactive that cumbersome procedures become necessary to insure stability during storage. The polyether chains to be used in the present invention have desirably molecular weights ranging from 200 to 20,000. Below 200, the nature of individual blocks in the polyether-polyamide copolymer produced deteriorates to reduce its blood compatibility. Above 20,000, the molecular weight of the polyether chains is so high that their reactivity deteriorates to introduce difficulty in copolymerization.

The polyether-polyamide block copolymer to be used in the present invention has desirably a molecular weight ranging from 7,000 to 100,000, with the range 10,000 - 50,000 being desirable. Below 7,000, the copolymer will not have satisfactory mechanical properties; beyond 100,000, the copolymer is unduly viscous and causes difficulty in handling. The content of polyether chains in the polyether-polyamide block copolymer under consideration ranges desirably from 2 to 50, preferably from 3 to 50 wt%. Below 2 wt%, the blood compatibility of the block copolymer is undesirably low; beyond 50 wt%, the block copolymer will have undesirably low mechanical strength.

In the case of the polymer alloy, each of the block copolymer and polyamide has desirably a molecular weight of 5,000 - 100,000, more desirably 10,000 - 50,000. Below 5,000, the polymer alloy of the block copolymer and the polyamide will not have satisfactory mechanical properties and problems may arise in terms of the matter that dissolves out. Beyond 100,000, the polymer alloy will become unduly viscous to cause difficulty in handling.

There is no particular limitation on the content of polyether in the polymer alloy under consideration; desirably, when blended with the polyamide, the polyether is contained in an amount of 0.5 - 60, preferably 3-50 wt% of the whole polymer alloy. Beyond 60 wt%, the mechanical and other properties of the polymer alloy will deteriorate to undesirable levels. In order for ordinary polyether nylons to manifest blood compatibility, the polyether content is desirably 5 - 15 wt% and more. In contrast, the polymer alloy of the present invention will exhibit satisfactory blood compatibility even if the polyether is present at lower concentrations and this is because depending on the method of shaping or molding, the polyether-polyamide block copolymer is concentrated on the blood-contacting surface. While the polymer alloy under discussion has good blood compatibility, it is particularly characterized by less adhesion of platelets to its surface. The criterion for evaluating the compatibility of the polymer alloy with platelets will be described later in this specification in the section of Examples together with specific procedures; to state briefly, the criterion is the number of platelets that adhere to the surface of the polymer alloy. In the case of ordinary polymeric materials such as polypropylene, polystyrene and nylons, several hundred to a thousand platelets will adhere to the surface; in contrast, less than a hundred platelets will adhere to the surface of the block copolymer or polymer alloy under discussion and this suggests that the activation of platelets which is a critical factor in blood coagulation has been suppressed to a low level.

It is economically advantageous for most of the medical devices that contact blood to be manufactured by melt molding and it is also preferred for the blood compatible materials of the present invention to be moldable in a molten state. Stated more specifically, aliphatic polyamides that have aromatic or aliphatic rings in the backbone chain are desirable since they are not only soluble in organic solvents but also moldable in a molten state.

However, block copolymers and polymer alloys thereof that are derived from wholly aromatic polyamides as typified by phenylene isophthalamide and phenylene terephthalamide are known to be substantially incapable of molding in a molten state since the wholly aromatic polyamides from which they originate have no big differences between melting point (400 - 500°C) and heat decomposition point (400 - 450°C) and because the block copolymers or polymer alloys thereof have glass transition points of about 300°C (see "Handbook of Polyamide Resins", Nikkan Kogyo Shinbunsha, 1988). Hence, the block copolymers and polymer alloys thereof that are derived from wholly aromatic polyamides are not thermoformable and are limited in terms of the methods of preparation and molding and their use.

As for the block copolymer under consideration or for the block copolymer or polyamide in the polymer alloy also under consideration, it is optional that 5 - 100 mol% of all terminal groups are capped with hydrocarbon groups having 1 - 22 carbon atoms. This may sometimes be effective in improving the thermal stability of the block copolymer or polymer alloy. Capping hydrocarbon groups are those which have 1 - 22 carbon atoms, preferably 6 - 22 carbon atoms. Specific examples include: aliphatic hydrocarbon groups such as methyl, ethyl, propyl, butyl, hexyl, octyl, nonyl, undecyl, dodecyl, heptadecyl, octadecyl, eicosyl and docosyl; alicyclic hydrocarbon groups such as cyclohexyl, alkylcyclohexyl and cyclohexylalkyl; and aromatic hydrocarbon groups such as phenyl, toluyl, naphthyl, benzyl and β-phenylethyl. These terminal capping hydrocarbon groups can be introduced into the polyether-polyamide block copolymer or polymer alloy thereof by producing them using monocarboxylic acids and/or monoamines that have such capping hydrocarbon groups.

The block copolymer under consideration or the block copolymer or polyamide in the polymer alloy also under consideration can be synthesized by ordinary methods of polyamide synthesis, as exemplified by interfacial polycondensation using the Shoten-Baumann reaction, melt polycondensation, and solution polycondensation. Melt polycondensation would be the best method from an industrial viewpoint.

The polymer alloy under consideration can be prepared by known methods of producing polymer alloys, such as melt blending the block copolymer with the polyamide in a single- or twin-screw extruder, or mixing and dissolving the two components in a solvent and causing reprecipitation of the mixture. The latter method which proceeds via solution is particularly advantageous in the case where the produced polymer alloy is to be used either as a coating material or in a selective separating membrane.

In the case of performing the melt polycondensation of polyether polyamide, a reaction system in which the polyether and polyamide components have low compatibility with each other will experience phase separation between the two components as the degree of polymerization increases, thereby yielding the desired polymer alloy.

Thus, the polymer alloy under consideration need not always be a compatible system and it may be a noncompatible system if there is no problem with mechanical strength.

The blood compatible materials that use the block copolymer or polymer alloy that have been described above can be employed in medical devices such as permselective membranes that are typified by artificial kidneys. Such permselective membranes in artificial kidneys are used as membranes in filtration or dialysis that permit selective permeation incase of uremia; when permitting the passage of blood, the membranes have preferably a sieving coefficient (SC) for albumin of 1% or less, more preferably 0.8 % or less. The reason for this is excess amount of albumin is harmful to the body.

Permselective membranes can be produced by any known methods, which include the dry process, the wet coagulation process and the "dry-wet process" which is the combination of the dry and wet processes. In the dry process, the block copolymer or polymer alloy of interest is dissolved in a suitable solvent and the resulting solution is cast onto a smooth flat sheet such as a glass plate, followed by distilling off the solvent. In the wet coagulation process, the block copolymer is immersed in a non-solvent (poor solvent) so as to yield a polymer deposit. One of these methods can be used to produce hollow-fiber membranes. Examples of the solvent that dissolve the block copolymer or polymer alloy of interest include dimethyl sulfoxide, N-methylpyrrolidone, dimethylformamide, dimethylacetamide, N,N'-dimethylimidazolidinone, cresol, hexafluoropropanol, formic acid, sulfuric acid, methanesulfonic acid and phosphoric acid. Unlike the conventional aliphatic polyether polyamides, the block copolymer and polymer alloy to be used in the present invention can be dissolved in common organic solvents and this is one of their major characteristic features. The block copolymer (I) or polymer alloy thereof (II) has preferably a concentration of 5 - 40 wt%, more preferably 5-30, 10 - 30, most preferably 10-25 wt%, in solution. It is optional to mix the above-listed solvents with organic or inorganic compounds that are conventionally used in the production of reverse osmotic and ultrafiltration membranes, as exemplified by calcium chloride, lithium chloride, magnesium chloride, magnesium perchlorate, urea and urea derivatives. The coagulation bath to be used in the practice of the wet coagulation process may be comprised of: water; alcohols such as methanol, ethanol, propanol, ethylene glycol and glycerin; acetone and dioxane; mixed solutions of these solvents; as well as mixed solutions that are prepared by adding non-solvents (poor solvents) to those solvents which dissolve the block copolymer (I) or polymer alloy (II), thereby lowering the solubilizing power of those solvents. It is also optional to add and dissolve a

6

third component in the coagulating solution and examples of the third component are organic and inorganic compounds such as calcium chloride and urea.

The mechanism by which the blood compatible materials of the present invention exhibit excellent biocompatibility as typified by strong resistance to thrombosis would be such that a microphase separation structure that comprises polyamide and polyether segments is formed on the surface of the blood compatible material, thereby permitting the latter to manifest good anti-thrombotic quality.

Because of their good blood compatibility, the block copolymer and polymer alloy under consideration are used in medical devices that contact blood and specific examples of such blood contacting medical devices include membranes for oxygenator and kidneys, various kinds of catheters, various kinds of tubes such as blood circuits, blood bags, various kinds of blood filters, and artificial blood vessels.

In view of their major feature of comprising a polyamide having a heat of crystallization of no more than 30 mJ/mg, the blood compatible materials of the present invention can be shaped after being dissolved in polar organic solvents such as DMSO, DMAc and NMP. The thus prepared solutions may preferably be coated on the surface of substrates that need be provided with resistance to thrombus formation, or the solutions may be put into non-solvents (poor solvents) to produce membranes by the wet process.

The hollow-fiber membrane of the present invention is produced by the following procedure: a spinning solution and a coagulating fluid are extruded simultaneously through a spinneret by means of a gear pump, with the spinning solution in the outer tube of the spinneret and the coagulating fluid in the inner tube; when the spinning solution contacts the coagulating fluid, the polymer is deposited and gelation starts. The hollow shape that starts to gel runs in air and enters a coagulation tank. Preferably, the gelation of the hollow shape ends while it is falling in air before entering the coagulation tank.

The hollow fiber emerging from the coagulation tank passes through a water washing tank (40°C) and a dryer (60°C) before it is wound up onto a bobbin by means of a takeup machine.

The spinning solution (polymer solution) is prepared by dissolving the polyether-polyamide block copolymer or polymer alloy of the present invention in a polar solvent. Exemplary polar solvents include dimethyl sulfoxide (DMSO), N,N-dimethylacetamide (DMAc), N,N-dimethylformamide (DMF) and N-methylpyrrolidone (NMP), which may be used either alone or as admixtures in solution. The respective solvents are different in their ability to mix with water or dissolve the polymer, thereby affecting various factors such as the viscosity of the polymer solution and the rate of polymer deposition from the polymer solution as immersed in water. With the polymer of interest, DMSO or mixed solutions thereof are preferably used as solvents.

The polymer solution has preferably a concentration of 5 - 40 wt%. Below 5 wt%, the strength of the hollow fiber is insufficient to prevent troubles such as leakage. Above 40 wt%, an excessively dense structure will result, making it impossible to provide satisfactory permeability to solutes.

Coagulants used for coagulating fluids are not limited to any particular agents. Examples of coagulants may include water, methanol, ethanol, propanol, ethyleneglycol, the solvent which solve or swell the block copolymer or alloy of the present invention, and the mixture of the solvent and water.

The structure of the hollow fiber to be produced from the polymer solution under consideration can be modified by adding another component to the polymer solution. Stated more specifically, a hollow-fiber membrane as produced from a spinning solution that comprises the polymer of interest and a solvent will have at least an active layer which is located on inner surface or outer surface of the hollow-fiber, a fingerlike void portion and a porous portion which is located between the active layer and the opposite side of the active layer. The overall structure of this membrane is generally called a "finger structure" and it is capable of permitting the passage of high volume of water.

In contrast, a hollow-fiber membrane as produced from a spinning solution that comprises the polymer of interest, a solvent and another water-soluble component has at least an active layer, which is located on inner surface or outer surface of the hollow-fiber, and a porous portion that is located inner or outer side to the active layer and which has a microporous structure with continuous open pores. The overall structure of this membrane is generally called a "sponge structure" and it is capable of efficient permeation of various solutes including urea, creatinine and vitamin $B_{12}$.

The solvent to be used in forming membranes having a sponge structure is preferably a polar solvent such as DMSO, DMAC, DMF or NMP which is used in amounts of 40 - 93 wt % based on the spinning solution.

The water-soluble component to be used in forming membranes having a sponge structure are exemplified by water-soluble polymeric substances such as polyethylene glycol, polyvinyl pyrrolidone and methyl cellulose, alcohols such as methyl alcohol and ethyl alcohol, and low-molecular weight substances such as glycerin.

Addition of an inorganic salt such as, calcium chloride, lithium chloride, magnesium chloride, magnesium perchlorate or another additive such as urea can be used in the spinning solution. The additive can be used likewise the water-soluble component.

These water-soluble components are added in amounts that vary with the specific kind to be added, their molecular weight, and the concentration of the polyetherpolyamide block copolymer or polymer alloy thereof. They are preferably added in amounts of 2 - 20 wt%. Below 2 wt%, no sponge structure will form; above 20 wt%, dissolution or deposition may occur. Another problem that can occur in the case of adding the water-soluble components in large volumes is difficulty in washing.

Polyvinyl pyrrolidone (PVP) has good compatibility with the block copolymer or polymer alloy thereof under consideration. If PVP of high molecular weight ($\overline{M}n$ = 36,000) is added, the viscosity of the spinning solution increases and, from a practical viewpoint, the addition of such PVP should desirably not exceed 6 wt%. In case of PVP having a low molecular weight ($\overline{M}n$ 10,000), the addition should desirably not exceed 15 wt%.

The additives mentioned above also contribute to the control of pore size and may be added as pore-size control agents.

The hollow-fiber membrane of the present invention is preferably used for hemodialysis and, hence, is required to be free from the leakage of albumin which is an effective component of blood. The sieving coefficient (SC) for albumin of the hollow-fiber membrane of the present invention is no more than 1% and this will cause no problems in practice. Compared to the conventional hollow-fiber dialysis membranes which are formed of regenerated cellulose, the hollow-fiber membrane of the present invention permits efficient passage of water (it has an ultrafiltration rate of 2-1200 ml/m$^2$ hr. mmHg at 37°C) and exhibits high performance in allowing the passage of not only medium-molecular weight substances such as vitamin $B_{12}$ (Mw = 1355) but also low-molecular weight proteins such as $\beta_2$-Mg (Mw = 11,600) which is believed to be a causative substance of dialytic amyloidosis.

## EXAMPLES

The following examples are provided for the purpose of further illustrating the present invention but are in no way to be taken as limiting. The various physical properties and membrane performance factors that are described hereinabove were determined by the following methods of measurement and testing.

(Characteristics of polymers)

i) Heat of crystallization, $\Delta Hc$; melting temperature of crystal, Tm; and heat of melting of crystal, $\Delta Hm$

Sample polymers were subjected to differential thermal analysis (DTA) on a differential scanning calorimeter. Each sample polymer was heated to the melting point + 20°C or 250°C at a rate of 10°C/min, held at that temperature for 3 min, then cooled at a rate of 5°C/min. The resulting temperature changes were measured. As a result of the phase change, the specific heat of the sample varied and the gradual change in the specific heat was measured continuously. The quantity of heat that was generated upon crystallization of the sample was determined from the corresponding peak.

(Characteristics of flat-sheet membrane)

i) Water flow rate

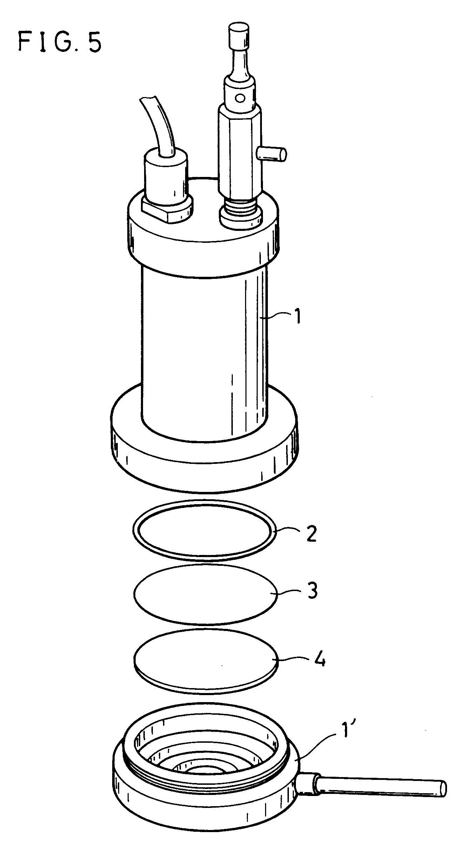

A flat-sheet membrane as prepared in accordance with the present invention was punched in a disk having a diameter of 43 mm and set up in a cell of the type shown in Fig. 5. The volume of distilled water that permeated through the membrane at 37°C under a pressure of 150 mmHg was determined for calculation of the water flow rate, ultrafiltration rate (UFR).

ii) Permeability to solutes

(Urea)

Distilled water (50 ml) and a urea solution (50 ml) having a concentration of 100 mg/dl were set in two compartments (1 and 2) of the cell in such a way that they contacted each other via the flat-sheet disk (43 mm$\phi$). The fluids in the respective compartments were agitated for 30 and 50 min. Thereafter, the volumes of fluids (V) in the two compartments and the concentrations of urea (C) in those compartments were determined by the urease indophenol method. On the basis of the determined values of V and C, the permeability to urea (P) of the flat-sheet membrane was determined by the following equation (1):

$$P = \frac{Ln[(\Delta C)_{t1}/(\Delta C)_{t2}]}{A(1/V_1 + 1/V_2)(t_2-t_1)} \text{ (cm/min)} \tag{1}$$

where

\* $(\Delta C)_{t1}$ and $(\Delta C)_{t2}$ = difference of solute concentration on both sides of the membrane at measuring times $t_1$ and $t_2$,

\* $V_1$ and $V_2$ = volume of solutes,

\* A = membrane area,

(Vitamin $B_{12}$)

The same procedure as described above was repeated except that the urea solution was replaced by a solution of vitamin $B_{12}$ having a concentration of 5 mg/dl and that the solute concentration was measured in terms of absorbance at 360 nm. The permeability to vitamin $B_{12}$ was calculated by equation (1).

iii) Test on platelet expanding ability

A sample of human platelet-rich plasma diluted to a platelet count of $10^5$ cells/$\mu$l was dropped on a flat-sheet membrane that had been prepared in accordance with the present invention; the membrane was then left to stand at room temperature for 30 min. Thereafter, the sample was washed with a phosphate buffer solution, fixed with glutaraldehyde and freeze-dried. The surface of the flat-sheet membrane was examined with a scanning electron microscope (SEM) and five fields of picture were taken at a magnification of 1,000. On the basis of the pictures taken, the platelets that adhered to the surface of the flat-sheet membrane were classified morphologically and the number of adhering platelets was counted.

(Morphological classification)

Type I: Non-active adhesion

(Ia) Normal disk shape.
(Ib) Spheroidized but not so deformed as to extend pseudopodia.

Type II: Active adhesion

Adhered with extending pseudopodia.

(Characteristics of hollow-fiber membrane)

i) Measuring water flow rate, ultrafiltration rate (UFR)

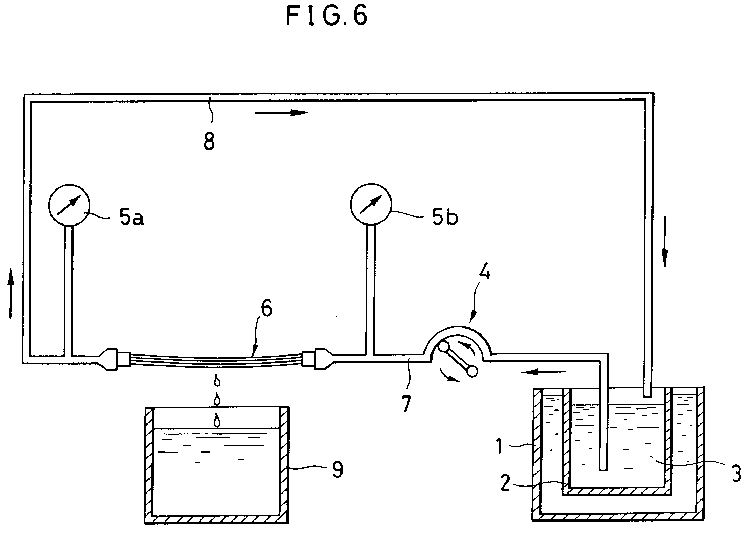

As shown in Fig. 6, a minimodule 6 fitted with a hollow-fiber membrane was connected between tubes 7 and 8, with a rotary pump 4 being fitted on the tube 7. Pressure gauges 5a and 5b were installed near opposite ends of the module 6. A container 2 of physiological saline 3 that had been warmed at $37 \pm 1°C$ was submerged in a thermostatic bath 1. A measuring circuit was constructed by submerging the tip of the tube 7 in the physiological saline 3 and connecting an end of the tube 8 to the container 2. The rotary pump 4 was set in such a way as to provide a linear velocity of 100 cm/min (flow rate/cross-sectional area) at the input end and a pressure difference of 100 mmHg across the membrane; the physiological saline was circulated under these conditions. When a steady state was reached, the permeant physiological saline was collected in a receptacle 9. The relationship between the permeation of physiological saline and time was determined and divided by the area of the membrane to calculate the flow rate of physiological saline (ml/m$^2 \cdot$ hr $\cdot$ mmHg). ii) Permeability to solutes

(Measuring the permeation coefficient of vitamin $B_{12}$)

In accordance with the teaching of High Performance Membrane 89', vol. 27, p. 54, Tokyo Igaku Shuppan, the procedure processed that the time-dependent change in the absorbance of laser light at 543 nm was measured with a silicon photodiode to determine the concentration of vitamin $B_{12}$ in the hollow-fiber membrane.

(Measuring the sieving coefficient (SC) for albumin)

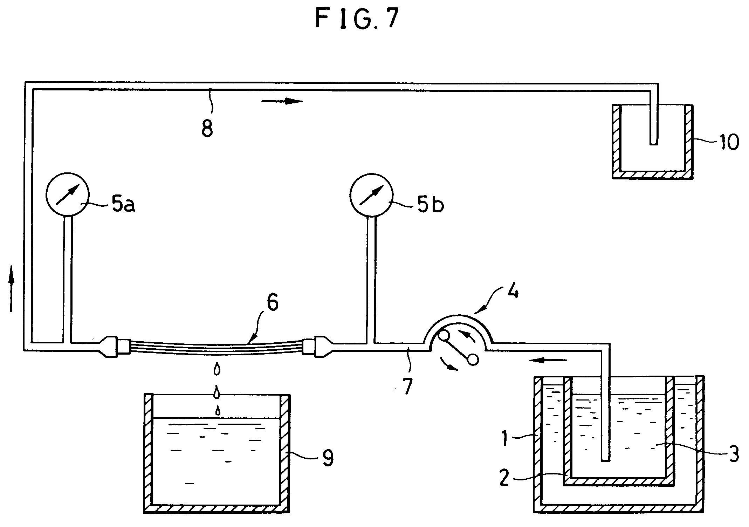

As shown in Fig. 7, the measuring circuit shown in Fig. 6 was modified in such a way that the output end was connected to a waste sump, whereby the circuit formed a single path. The container 2 was filled with a sample of bovine blood (warmed at $37 \pm 1°C$) that had been conditioned to a hematocrit count of 25% and a total protein content of about 6.0 g/dl. The rotary pump 4 was so operated that the blood sample would flow at a linear velocity of 100 cm/min at the input end of the module 6 to produce a pressure difference of 100 mmHg across the membrane. After a steady state was reached, fluids were collected at the input and output ends of the module 6 and the filtrate (permeant) was also collected. The concentrations of the respective fluids and filtrate were determined by the BCG (bromocresol green) method and the sieving coefficient (SC) for albumin was calculated by the following equation (2):

$$SC = C_F/\{(C_{Bi} + C_{B0})/2\} \qquad (2)$$

where

$C_F$:  the concentration of the filtrate;
$C_{Bi}$:  the concentration of fluid at the input end of the module;
$C_{B0}$:  the concentration of fluid at the output end of the module.

(Molecular weight dependency of SC as determined on aqueous solutions of dextran)

Dextran T10 and T40 (Pharmacia, average molecular weight: 10,000 for T10 and 40,000 for T40) were each dissolved in physiological saline at a concentration of 10 g/L and pumped at a linear velocity of 100 cm/min to produce a filtration pressure of 100 mmHg in the same column and detector equipment as used for measuring the SC for albumin. Fluids were collected at the input and output ends of the module and the filtrate was also collected. Using a high-performance liquid chromatograph (HPLC of Showa Denko K.K.; column, OHpak SB-803 x 2; detector, Shodex GPC System-11), the concentrations of the respective fluids and filtrate were measured and the SC values for the respective molecular weights were determined by equation (2).

(Synthesis of the polyether-polyamide block copolymer, production of polymer alloy using the same, and making membranes therefrom)

In order to produce the polyether-polyamide block copolymer which is to be used in the present invention, a polyamide that is prepared by polycondensation of a diamine and a dicarboxylic acid is subjected to condensation reaction with a diaminopolyethylene glycol or dicarboxylic acid polyethylene glycol, thereby binding the two compounds through amide bonds. This may be accomplished as follows. First, sebacic acid (404 g) is dissolved in ethanol (2200 ml) under heating. To the solution, metaxylylenediamine (272 g) as dissolved in ethanol (400 ml) is added and the ingredients are mixed under stirring, whereupon nylon MXD10 salt will be deposited. This nylon MXD10 salt was filtered, dried and dissolved in water to make a 1% aqueous solution, which was found to have a pH of $7.5 \pm 0.5$. The nylon MDX10 salt (22.5 g) and polyethylene glycol (2.5 g) having an amine group at both ends and whose molecular weight was 2000 were dissolved in ion-exchanged water (40 ml) in a test tube under heating to prepare a uniform solution. The test tube was set up in an autoclave, purged with nitrogen and heated. When the pressure in the autoclave rose to 10 kgf/cm$^2$, water was gradually distilled to maintain the pressure. When water was no longer evaporable, reaction was carried out at 240°C for 4 h at a pressure of 10 kgf/cm$^2$; subsequently, the autoclave was slowly depressurized to vacuum. Finally, reaction was carried out at 240°C for 4 h at a reduced pressure of 10 mmHg to yield the end polymer. The resulting polyether-polyamide block copolymer was dissolved in formic acid and reprecipitated in distilled water or a mixed solution of methanol/water for subsequent membrane making or alloying.

Another method may proceed as follows. First, isophthalic acid (I) (1060 g), adipic acid (15 g), 1,3-bisaminomethylcyclohexane (B) (617 g), hexamethylenediamine (6) (237 g), polyethylene glycol (PEG) (200 g) terminating with an amino group at both ends and having a molecular weight of 2000, and water (3000 g) were charged into an autoclave equipped with a stirrer; the autoclave was then purged with nitrogen and heated. When the pressure in the autoclave rose to 19 kgf/cm$^2$, water was gradually distilled to maintain the pressure. When water was no longer evaporable, reaction was carried out at 240°C for 4 h at a pressure of 10 kgf/cm$^2$; subsequently, the autoclave was slowly depressurized as the temperature was elevated to 260°C. In the final stage, the pressure was brought to a vacuum of 20 mmHg over 120 min; thereafter, the pressure was reverted to one atmosphere and the end polymer, PEG-NyBI/6I (7/3), was recovered. This polyether-polyamide block copolymer was dissolved in formic acid and reprecipitated in distilled water or a mixed solution of methanol/water for subsequent membrane making or alloying.

A film was made from a polymer alloy of PEG-NyBI and NyBI by the following method in accordance with the present invention.

First, polyethylene oxide-polyiminomethylene-1,3-cyclohexylmethyleneiminoisophthaloyl (PEG-NyBI) was synthesized by the following procedure. Isophthalic acid (I) (1059 g) and 1,3-bisaminomethylcyclohexane (B) (821 g) were dissolved in water (2000 g) and charged into an autoclave equipped with a stirrer. The autoclave was then purged with nitrogen and heated. When the pressure in the autoclave rose to 15 kgf/cm$^2$, water was distilled with the pressure maintained at that level. When water was no longer evaporable, reaction was carried out at 250°C for 2 h at a pressure of 13 kgf/cm$^2$. Subsequently, polyethylene oxide (600 g) that was terminated with an amino group at both ends and which had a molecular weight of 1000 was injected under pressure into the reaction system and reaction was continued for an additional 2 h. Thereafter, the autoclave was slowly depressurized and reaction was further carried out for 15 min at a reduced pressure of 450 mmHg. Following pressurization with nitrogen, the end polyether-polyamide block copolymer, PEG (30%)-NyBI, was recovered.

In the next place, polyimino-1,3-methylenecyclohexylmethyleneiminoisophthaloyl (NyBI) was synthesized in basically the same manner as described above.

Further, both PEG-NyBI and NyBI were dissolved in DMSO to make 20 wt% solutions, which were mixed in predetermined proportions, cast onto a glass plate, and vacuum dried to distill off DMSO, thereby producing a polymer alloy film.

Examples 1 - 22

Various grades of polyether-polyamide block copolymer containing polyamides that had ΔHc values of no more than 30 mJ/mg (see Table 1) were dissolved in various solvents (see Table 2-1 and Table 2-2) at temperatures ranging from room temperature to 100°C, thereby preparing doping solutions each having a polymer concentration of 20 wt%. The thus prepared doping solutions were left to stand for 2 h to effect defoaming. The defoamed doping solutions were cast in a predetermined thickness onto glass plates in a vapor phase. Immediately thereafter, the cast films were coagulated by 5-min immersion in coagulants (for their composition, see Table 2-1 and Table 2-2) that had been conditioned to a temperature of 5°C. Subsequently, the residual organic solvents in the coagulated films were removed by washing under running water to yield wet membranes, which had thicknesses of 30 - 80 μm in a wet state. The respective membranes were checked for water flow rate and permeabilities to urea and vitamin B$_{12}$.

The compositions of the polyether-polyamide block copolymers that were prepared in Examples 1 - 22 and their permeation performance are shown in Table 2-1 and Table 2-2.

Table 1

| Polyamide (wt% ratio) | ΔHc (mJ/mg) | Tm (°C) | ΔHm (mJ/mg) | Solubility in DMSO |
|---|---|---|---|---|
| Nylon MXD10 | 0 | 178 | 16 | soluble |
| Nylon MXD10/69 (9/1) | 0 | 176 | 26 | soluble |
| Nylon MXD10/69 (7/3) | 0 | 147 | 20 | soluble |
| Nylon) MXD10/69 (5/5) | 0 | 131 | 20 | soluble |
| Nylon 6I/66 (45/55) | 17 | 191 | 16 | soluble |
| Nylon 6I/610 (4/6) | 20 | 174 | 24 | soluble |
| Nylon 6T/6I/610 (25/15/60) | 28 | 183 | 19 | soluble |
| Nylon 6I/610 (7/3) | 0 | - | - | soluble |
| Nylon BI | 0 | 256 | 15 | soluble |
| Nylon BI/6I (7/3) | 0 | - | - | soluble |
| Nylon B10 | 0 | 180 | 16 | soluble |
| Nylon BI/B10 (5/5) | 0 | - | - | soluble |

Table 2-1

| | Block Copolymer | | Solvent of Dope Composition (Polymer Concentration wt%) | Coagulant Composition = wt% ratio | Permeability | | |
|---|---|---|---|---|---|---|---|
| | [1]PEG (wt%) | Polyamide (wt% ratio) | | | [2]UFR | [3]Urea | [3]VB12 |
| Ex. 1 | 10 | [4]Nylon MXD10 | [5]DMSO (20) | [6]M/H=3/7 | 51 | – | 82 |
| Ex. 2 | 10 | Nylon MXD10 | Formic Acid (22) | M/H=7/3 | 66 | – | 91 |
| Ex. 3 | 10 | [17]Nylon MXD10/69 (9/1) | Formic Acid (22) | M/H=3/7 | 31 | 391 | 77 |
| Ex. 4 | 10 | Nylon MXD10/69 (7/3) | Formic Acid (22) | M/H=5/5 | 25 | – | 79 |
| Ex. 5 | 10 | Nylon MXD10/69 (5/5) | Formic Acid (22) | M/H=3/7 | 40 | – | 77 |
| Ex. 6 | 5 | Nylon MXD10 | DMSO (20) | M/H=9/1 | 34 | – | 67 |
| Ex. 7 | 20 | Nylon MXD10 | DMSO (16) | M/H=7/3 | 47 | – | 76 |
| Ex. 8 | 40 | Nylon MXD10 | DMSO (20) | M/H=1/9 | 23 | – | 65 |
| Ex. 9 | 10 [7]PEG 400] | Nylon MXD10 | DMSO (16) | M/H=7/3 | 55 | – | 84 |
| Ex. 10 | 10 [8]PEG 4000] | Nylon MXD10 | DMSO (14) | M/H=1/9 | 21 | – | 45 |
| Ex. 11 | 10 [9]PPG 2000] | Nylon MXD10 | DMSO (16) | M/H=1/9 | 16 | – | 53 |
| Ex. 12 | 10 [10]PTMG 2000] | Nylon MXD10 | DMSO (16) | M/H=3/7 | 37 | – | 66 |
| Ex. 13 | 10 | Nylon 6 [11]I/66 (45/55) | DMSO(16) + [13]CaCl2 | M/H=1/9 | 40 | – | 64 |
| Ex. 14 | 10 | [18]Nylon 6 [12]T/6I/610 (25/15/60) | Formic Acid (22) | M/H=1/9 | 20 | – | 51 |
| Ex. 15 | 10 | Nylon MXD10/69 (9/1) | DMSO(16) + [14]Urea | M/H=3/7 | 98 | – | 89 |
| Ex. 16 | 10 | Nylon 6I/610 (7/3) | DMSO (17) | M/H=3/7 | 70 | – | 59 |
| Ex. 17 | 10 | Nylon [15]BI/6I (7/3) | DMSO (20) | M/H=6/4 | 120 | – | 74 |

EP 0 600 793 B1

Table 2-2

| | Block Copolymer | | Solvent of Dope Composition (Polymer Concentration wt%) | Coagulant Composition = wt% ratio | Permeability | | |
|---|---|---|---|---|---|---|---|
| | PEG (wt%) | Polyamide (wt% ratio) | | | [2]UFR | [3]Urea | [3]VB12 |
| Ex. 18 | 10 | Nylon BI/6I (7/3) | DMSO (20) | [16]DMSO/H=7/3 | 27 | – | 73 |
| Ex. 19 | 10 | Nylon BI/6I (9/1) | DMSO (20) | DMSO/H=7/3 | 35 | – | 69 |
| Ex. 20 | 10 | Nylon BI | DMSO (20) | M/H=6/4 | 132 | – | 80 |
| Ex. 21 | 10 | Nylon B10 | DMSO (20) | M/H=6/4 | 80 | – | 55 |
| Ex. 22 | 10 | Nylon BI/B10 (5/5) | DMSO (20) | M/H=6/4 | 72 | – | 63 |
| C.Ex. 1 | 25 [PPG 2000] | Nylon 12 | Formic Acid (22) | M/H=5/5 | 31 | – | 11 |
| C.Ex. 2 | 10 | Nylon 12 | Formic Acid (22) | M/H=4/6 | 30 | – | 25 |
| C.Ex. 3 | 10 | Nylon 6 | Formic Acid (22) | M/H=3/7 | 43 | – | 17 |
| C.Ex. 4 | 10 | Nylon 66 | Formic Acid (22) | M/H=1/9 | 121 | – | 33 |
| C.Ex. 5 | 10 | Nylon 610 | Formic Acid (22) | M/H=3/7 | 24 | 344 | 34 |
| C.Ex. 6 | 10 | Nylon MXD6 | Formic Acid (22) | M/H=3/7 | 7 | – | 13 |
| C.Ex. 7 | 50 [PTMG 2000] | Nylon 12 | Formic Acid (22) | M/H=5/5 | 25 | – | 9 |
| C.Ex. 8 | 25 [PPG 2000] | Nylon 6 | Formic Acid (22) | M/H=3/7 | 21 | – | 6 |
| C.Ex. 9 | 10 | Nylon 66/610 (1/2) | Formic Acid (22) | M/H=3/7 | 6 | 230 | 34 |

Notes)

1)    PEG: polyethylene glycol having amine at both ends and a molecular weight of 2000

2)    Unit: ml·hr·mmHg·m$^2$

3)    Unit: $10^{-4}$ cm/min

4)    Nylon MXD 10: polyamide comprising methaxylylenediamine and sebacic acid

5)    DMSO: dimethylsulfoxide

6)    M/H: methanol/water

7)    PEG 400: polyethylene glycol having amine at both ends and a molecular weight of 400

8)    PEG 4000: polyethylene glycol having amine at both ends and a molecular weight of 4000

9)    PPG 2000: polypropylene glycol having amine at both ends and a molecular weight of 2000

10)   PTMG 2000: polytetramethylene glycol having amine at both ends and a molecular weight of 2000

11)   I: isophthalic acid

12)   T: terephthalic acid

13)   added in 50% of the polymer weight

14)   added in 100% of the polymer weight

15)   B: 1,3-bisaminomethylcyclohexane

16)   DMSO/H: dimethylsulfoxide/water

17)   Nylon MXD 10/69 is a random copolymer comprising methaxylylenediamine and sebacic acid, hexamethylenediamine and azelaic acid, in weight ratio of 9:1.

18)   Nylon 6T/6I/610 is a random copolymer comprising hexamethylenediamine and terephthalic acid,

hexamethylenediamine and isophthalic acid,

hexamethylenediamine and sebacic acid, in weight ratio of

25:15:60.

Comparative Examples 1 - 9

Comparative samples of polyether-polyamide block copolymer were prepared from polyether and polyamides that had $\Delta$Hc values as shown in Table 3. The permeabilities of the membranes that were made from those block copolymers were measured as in Examples 1 - 22. The results are shown in Table 2-2.

Table 3

| Polyamide wt% ratio) | $\Delta$Hc (mJ/mg) | Tm (°C) | Solubility in DMSO |
|---|---|---|---|
| [1]Nylon 6 | 50 | 225 | insoluble |
| [2]Nylon 66 | 56 | 264 | insoluble |
| [3]Nylon 610 | 49 | 226 | insoluble |
| [4]Nylon 12 | 57 | 181 | insoluble |
| [5]Nylon 66/610 (1/2) | 42 | 194 | insoluble |
| [6]Nylon MXD 6 | 36 | 243 | insoluble |

Notes)
1) polyamide of $\varepsilon$-caprolactam
2) polyamide of hexamethylenediamine and adipic acid
3) polyamide of hexamethylenediamine and sebacic acid
4) polyamide of lauric lactam (dodecanoic lactam)
5) random copolymer comprising hexamethylenediamine and adipic acid, hexamethylenediamine and sebacic acid, in weight ratio of 1:2
6) polyamide of methaxylylenediamine and adipic acid

As is clear from the data shown in Table 2-1 and Table 2-2, the permselective membranes that were prepared in Examples 1 - 22 of the present invention were remarkably improved in their permeability to medium- and low-molecular weight solutes over the samples of Comparative Examples 1 - 9 that were conventionally known aliphatic polyether-polyamide block copolymers which had $\Delta$Hc values of more than 30 mJ/mg. The samples of Examples 1 - 22 were also improved in water permeation (UFR). In addition, the permeation characteristics of these samples could be readily controlled by varying the composition of the coagulant; hence, it was possible to prepare permselective membranes that had permeation performance suitable for a specific object.

Examples 23 - 30

Additional samples of polyether-polyamide block copolymer were prepared in accordance with the present invention and their blood compatibility was evaluated by a test on the platelet expanding ability. The results are shown in Table 4 together with the data for Comparative Examples 10 and 11.

Table 4

| | Block Copolymer | | Number of Adherent Platelet | | |
|---|---|---|---|---|---|
| | PEG (wt%) | Polyamide (wt% ratio) | Ia | Ib | II |
| C. Ex. 10 | 0 | Nylon 12 | 0 | 0 | 587 |
| C. Ex. 11 | 0 | Nylon MXD10 | 0 | 0 | 602 |
| Ex. 23 | 10 | Nylon MXD10 | 0 | 0 | 0 |
| Ex. 24 | 10 | Nylon MXD10/69 (9/1) | 0 | 0 | 2 |
| Ex. 25 | 10 | Nylon 6T/6I/610 (25/15/60) | 0 | 1 | 1 |
| Ex. 26 | 10 | Nylon 6I/610 (7/3) | 0 | 2 | 2 |
| Ex. 27 | 10 | Nylon BI/6I (7/3) | 0 | 1 | 1 |
| Ex. 28 | 10 | Nylon BI | 0 | 0 | 2 |
| Ex. 29 | 10 | Nylon B10 | 0 | 0 | 1 |
| Ex. 30 | 10 | Nylon BI/B10 (5/5) | 0 | 2 | 1 |

As is clear from Table 4, the permselective membranes that were prepared in Examples 23 - 30 of the present invention were improved over those of Comparative Examples 10 and 11 in that the adhesion of platelets was generally low. In particular, the active adhesion of type II was very low, indicating the high thrombotic resistance of those permselective membranes.

Examples 31 - 37

Various grades of polymer alloy according to the present invention (see Table 5) were dissolved in DMSO at room temperature to prepare doping solutions each having a polymer concentration of 20 wt%. The thus prepared doping solutions were left to stand for defoaming and cast in a predetermined thickness onto glass plates. Immediately thereafter, the cast films were coagulated by 5-min immersion in coagulants (for their composition, see Table 6) at 5°C. Subsequently, the residual organic solvents in the coagulated films were removed by washing under running water to yield wet membranes, which had thicknesses of 30 - 70 μm in a wet state. The respective membranes were checked for water flow rate and permeability to vitamin $B_{12}$.

Table 5

| | Block Copolymer | | Polyamide in Polymer Alloy (Copolymer wt/ Polyamide wt) | Solvent of Dope Composition (Polymer Concentration wt%) | Coagulant Composition = wt% ratio | Permeability | |
|---|---|---|---|---|---|---|---|
| | PEG (wt%) | Polyamide (wt% ratio) | | | | [7]UFR | [8]VB12 |
| Ex. 31 | 20 [1)]PEG 1000] | [4)]Nylon BI | Nylon BI (1/1) | DMSO (20) | M/H=6/4 | 115 | 65 |
| Ex. 32 | 20 [1)]PEG 1000] | Nylon BI | Nylon BI (1/1) | DMSO (20) | M/H=3/7 | 61 | 48 |
| Ex. 33 | 20 [1)]PEG 1000] | Nylon BI | Nylon BI (1/1) | DMSO (20) | DMSO/H=7/3 | 45 | 32 |
| Ex. 34 | 40 [2)]PEG 2000] | [5)]Nylon BI/6I (9/1) | Nylon BI/6I (9/1) (1/3) | DMSO (20) | M/H=6/4 | 132 | 70 |
| Ex. 35 | 40 [2)]PEG 2000] | Nylon BI/6I (9/1) | Nylon BI/6I (9/1) (1/3) | DMSO (20) | M/H=3/7 | 82 | 51 |
| Ex. 36 | 75 [3]PTMG 2100] | [6)]NylonBI/B10 (6/4) | Nylon BI/B10 (6/4) (1/2) | DMSO (20) | M/H=6/4 | 153 | 46 |
| Ex. 37 | 75 [3)]PTMG 2100] | Nylon BI/B10 (6/4) | Nylon BI/B10 (6/4) (1/2) | DMSO (20) | M/H=3/7 | 105 | 39 |

Notes)

1)    PEG 1000: polyethylene glycol having amine at both ends and a molecular weight of 1000

2)    PEG 2000: polyethylene glycol having amine at both ends and a molecular weight of 2000

3)    PTMG 2100: polytetramethylene glycol having amine at both ends and a molecular weight of 2100

4)    Nylon BI: polyamide of 1,3-bisaminomethylcyclohexane and isophthalic acid

5)    Nylon BI/6I (9/1): random copolymer comprising 1,3-bisaminomethylcyclohexane and isophthalic acid, hexamethylenediamine and isophthalic acid, in weight ratio of 9:1

6)    Nylon BI/B10 (6/4): random copolymer comprising 1,3-bisaminomethylcyclohexane and isophthalic acid, 1,3-bisaminomethylcyclohexane and sebacic acid, in weight ratio of 6:4

7)    Unit: $ml/hr \cdot mmHg \cdot m^2$

8)    Unit: $10^{-4}$ cm/min

Examples 38 - 41

Polymer alloy samples were prepared in accordance with the present invention using the various grades of polyether-polyamide block copolymer (I) and polyamide (II). The blood compatibility of these samples was evaluated by conducting a test on their ability to expand platelets. The results are shown in Table 6 below.

Table 6

| | | Block Copolymer | | Polyamide in Polymer Alloy | Solvent of dope solution | Coagulant Composition | Number of Adherent Platelets | | | |
|---|---|---|---|---|---|---|---|---|---|---|
| | | PEG (wt%) | Polyamide (wt% ratio) | (Copolymer wt/ Polyamide wt) | (Concentra- tion wt%) | | 10) | Ia | Ib | II |
| Ex. 38 | | 20 [1)PEG 1000] | 2)Nylon BI | Nylon BI (1/1) | 8)HFIP (5) | 9) – | A | 2 | 14 | 16 |
| | | | | | | | B | 0 | 1 | 2 |
| Ex. 39 | | 30 [1)PEG 1000] | Nylon BI | Nylon BI (1/2) | HFIP (5) | – | A | 0 | 0 | 1 |
| | | | | | | | B | 0 | 0 | 2 |
| Ex. 40 | | 40 [3)PEG 2000] | 4)Nylon BI/6I (9/1) | Nylon BI/6I (9/1) (1/3) | HFIP (5) | – | A | 0 | 0 | 0 |
| | | | | | | | B | 0 | 0 | 1 |
| Ex. 41 | | 75 [5)PTMG 2100] | 6)Nylon BI/B10 (6/4) | Nylon BI/B10 (6/4) (1/2) | HFIP (5) | – | A | 0 | 1 | 1 |
| | | | | | | | B | 0 | 1 | 4 |

EP 0 600 793 B1

Notes)

1) PEG 1000: polyethylene glycol having amine at both ends and a molecular weight of 1000

2) Nylon BI: polyamide of 1,3-bisaminomethylcyclohexane and isophthalic acid

3) PEG 2000: polyethylene glycol having amine at both ends and a molecular weight of 2000

4) Nylon BI/6I: random copolymer comprising 1,3-bisaminomethylcyclohexane and isophthalic acid, hexamethylenediamine and isophthalic acid in weight ratio of 9:1

5) PTMG: polytetramethylene glycol having amine at both ends and a molecular weight of 2100

7) Nylon BI/B10 (6/4): random copolymer comprising 1,3-bisaminomethylcyclohexane and isophthalic acid, a 1,3-bisaminomethylcyclohexane and sebacic acid in a weight ratio of 6:4

8) HFIP: hexafluoroisopropanol

9) dry process no coagulant (distilling off the solvent)

10) A, B: designate different donors

## Example 42

PEG 1000 (5)-NyBI (19 wt%) was dissolved in DMSO (81 wt%) at room temperature and the solution was filtered and left to stand under vacuum to effect defoaming. As a result, a stock solution for spinning by the wet process was prepared and it had a viscosity of 8.5 poises. This spinning solution and a coagulant (40°C) that consisted of 10 wt% DMSO and 90 wt% water (DMSO/$H_2$O = 1/9) were extruded through a spinneret simultaneously by means of a gear pump, with the spinning solution in the outer tube of the spinneret and the coagulant in the inner tube. The resulting hollow shape was caused to run in air and passed through a coagulating bath and a water washing tank to yield a hollow-fiber membrane having an inside diameter of 210 μm and a thickness of 25 μm.

## Examples 43 - 45

Hollow-fiber membranes each having an inside diameter of 210 μm and a thickness of 25 μm were prepared as in Example 42, except that the coagulant as supplied into the inner tube of the sheathed annular nozzle consisted of a

mixture of DMSO/$H_2$O at weight ratios of 2/8 (Example 43), 4/6 (Example 44) and 6/4 (Example 45).

Examples 46 - 50

As in Example 42, PEG 1000 (10%)-NyBI/6I (19 wt%) was dissolved in DMSO (81 wt%) and a stock solution for spinning by the wet process was prepared. This spinning solution had a viscosity of 17.6 poises at 30°C. Hollow-fiber membranes each having an inside diameter of 220 μm and a thickness of 30 μm were prepared as in Example 42, except that the following coagulants were supplied into the inner tube of the spinneret: water at 22°C in Example 46; a 2/8 mixture of DMSO/$H_2$O at 22°C in Example 47; a 2/8 mixture of DMSO/$H_2$O at 40°C in Example 48; a 4/6 mixture of DMSO/$H_2$O at 22°C in Example 49; and a 6/4 mixture of DMSO/$H_2$O at 22°C in Example 50.

Example 51

As in Example 42, a polymer alloy (19 wt%) that consisted of PEG 2000 (10%)-NyBI and NyBI was dissolved in DMSO (81 wt%) and a stock solution for spinning by the wet process was prepared. This spinning solution had a viscosity of 8.2 poises. A hollow-fiber membrane having an inside diameter of 220 μm and a thickness of 40 μm was prepared as in Example 42, except that a 4/6 mixture of DMSO/$H_2$O was supplied as a coagulant into the inner tube of the spinneret.

Example 52

PEG 1000 (5%)-NyBI (17 wt%) and PVP (12.75 wt%; Kollidon 30 K = 30; product of BASF; $\overline{M}n$ = 10,000) were dissolved in DMSO (70.25 wt%) at room temperature and the solution was filtered and left to stand under vacuum to effect defoaming. As a result, a stock solution for spinning by the wet process was prepared and it had a viscosity of 53 poises. This spinning solution and a coagulant (40°C) that was an 8/2 mixture of DMSO/$H_2$O were extruded through a spinneret simultaneously by means of a gear pump, with the spinning solution in the outer tube of the spinneret and the coagulant in the inner tube. The resulting hollow shape was caused to run in air and passed through a coagulating bath (DMSO/$H_2$O) and a water washing tank to yield a hollow-fiber membrane having an inside diameter of 210 μm and a thickness of 25 μm.

Example 53

As in Example 52, PEG 1000 (5%)-NyBI (18 wt%) and PVP (5.4 wt%; Kollidon 90 K = 90; product of BASF; $\overline{M}n$ = 360,000) were dissolved in DMSO (76.6 wt%) and a stock solution for spinning by the wet process was prepared. This spinning solution had a viscosity of 165.4 poises. A hollow-fiber membrane having an inside diameter of 210 μm and a thickness of 35 μm was prepared as in Example 52, except that a coagulant consisting of a 2/8 mixture of DMSO/$H_2$O was supplied into the inner tube of the spinneret.

Comparative Example 12

Commercial grade of regenerated cellulose hollow-fiber membrane (Regenerated Cellulose TE manufactured by Enka Co.) having an inside diameter of 200 μm and a thickness of 11 μm.

Example 54

The water flow rates (UFR) of the hollow-fiber membranes as prepared in Examples 42 - 53 are shown below together with the data for Comparative Example 12.

|  | UFR (ml/m2 • hr • mmHg) |
| --- | --- |
| Example 42 | 429 |
| Example 43 | 260 |
| Example 44 | 375 |
| Example 45 | 99 |
| Example 46 | 1175 |
| Example 47 | 752 |
| Example 48 | 485 |
| Example 49 | 125 |
| Example 51 | 354 |
| Example 52 | 134 |
| Example 53 | 59 |
| Comp. Ex. 12 | 4 |

### Example 55

The permeabilities to Vitamin $B_{12}$ of the hollow-fiber membranes as prepared in Examples 42, 52 and 53 are shown below together with the data for Comparative Example 12.

|  | Permeabilities to VB12 ($\mu$m/sec) |
| --- | --- |
| Example 52 | 3.04 |
| Example 53 | 2.97 |
| Example 42 | 1.56 |
| Comp. Ex. 12 | 0.78 |

### Example 56

The hollow-fiber membranes prepared in Examples 42 and 53 were measured for their SC values for albumin and the results are shown below.

|  | SC Value of Albumin (%) |
| --- | --- |
| Example 42 | 0.13 |
| Example 53 | 0.24 |

### Example 57

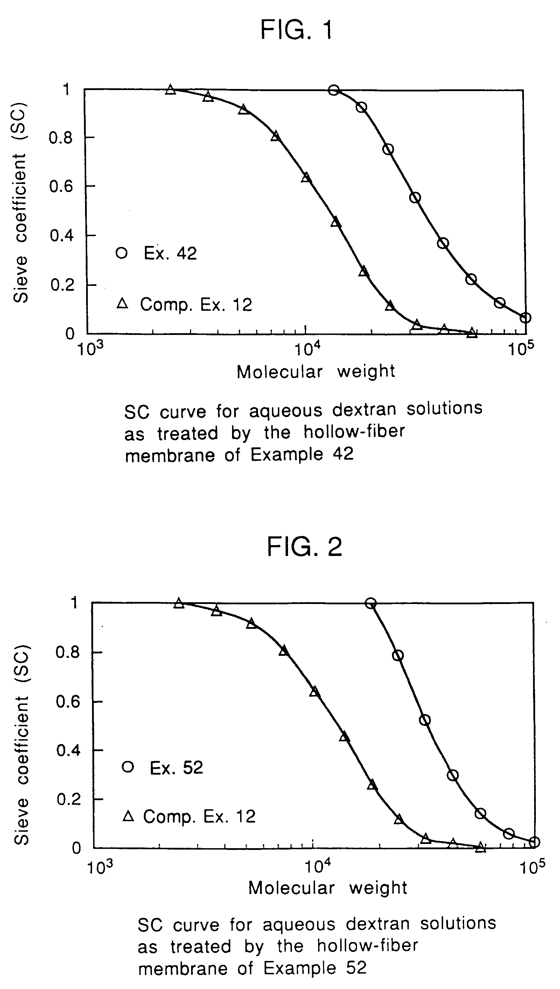

The SC values for the aqueous solution of dextran that was treated by the hollow-fiber membranes as prepared in Examples 42 and 52 were plotted against the molecular weight of dextran and the profiles are shown in Figs. 1 and 2, respectively, together with the SC curve for the sample of Comparative Example 12.

### Example 58

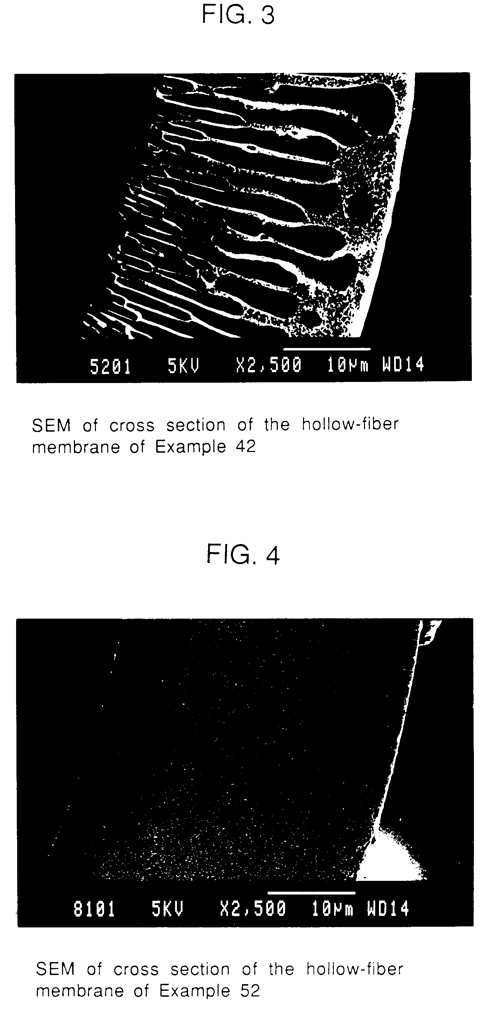

Scanning electron micrographs of cross sections of the hollow-fiber membranes as produced in Examples 42 and 52 are shown in Figs. 3 and 4, respectively.

The results of Examples 42-53 and Comparative example 12 are shown in Table 7-1 and Table 7-2.

Table 7-1

| | Block Copolymer | | Solvent of Dope Composition (Polymer Concentration wt%) | Coagulant Composition = wt% ratio (Temp.) | Permeability | | |
|---|---|---|---|---|---|---|---|
| | PEG (wt%) | Polyamide (wt% ratio) | | | [7]UFR | [8]VB12 | SC Value of Albumin (%) |
| [1),2)] Ex. 42 | 5 [[3] PEG 1000] | Nylon BI | DMSO (19) | DMSO/H=1/9 (40°C) | 429 | 1.56 | 0.13 |
| Ex. 43 | 5 [PEG 1000] | Nylon BI | DMSO (19) | DMSO/H=2/8 (40°C) | 260 | - | - |
| Ex. 44 | 5 [PEG 1000] | Nylon BI | DMSO (19) | DMSO/H=4/6 (40°C) | 375 | - | - |
| Ex. 45 | 5 [PEG 1000] | Nylon BI | DMSO (19) | DMSO/H=6/4 (40°C) | 99 | - | - |
| Ex. 46 | 10 [PEG 1000] | Nylon BI/6I | DMSO (19) | H (22°C) | 1175 | - | - |
| Ex. 47 | 10 [PEG 1000] | Nylon BI/6I | DMSO (19) | DMSO/H=2/8 (22°C) | 752 | - | - |
| Ex. 48 | 10 [PEG 1000] | Nylon BI/6I | DMSO (19) | DMSO/H=2/8 (40°C) | 485 | - | - |
| Ex. 49 | 10 [PEG 1000] | Nylon BI/6I | DMSO (19) | DMSO/H=4/6 | 125 | - | - |

Table 7-2

| | Block Copolymer | | Polyamide in Polymer Alloy (Other Component wt/ Polyamide wt) | Solvent of Dope Comp. (Polymer Concentration wt%) | Coagulant Composition = wt% ratio (Temp.) | Permeability | | |
|---|---|---|---|---|---|---|---|---|
| | PEG (wt%) | Polyamide (wt% ratio) | | | | [7]UFR | [8]VB12 | SC Value of Albumin (%) |
| Ex. 51 | 10 [[3] PEG 2000] | Nylon BI | [9] [5]Nylon BI (1/1) | DMSO (19) | DMSO/H=4/6 (22°C) | 354 | – | – |
| [1),2)] Ex. 52 | 5 [[4] PEG 1000] | Nylon BI | [10] [6]PVP | DMSO (19) | DMSO/H=8/2 (40°C) | 134 | 3.04 | – |
| Ex. 53 | 5 [[4] PEG 1000] | Nylon BI | [10] [6]PVP | DMSO (19) | DMSO/H=2/8( (40°C) | 59 | 2.97 | 0.24 |
| [1)] C.Ex.12 | Hollow-fiber membrane of regenerated cellulose | | | | | 4 | 0.78 | – |

24

Notes)

1)    SC VS molecular weight curves shown in Figs. 1 and 2

2)    Photomicrographs of a section of a film made of a hollow fiber shown in Figs. 3 and 4

3)    PEG 1000: polyethylene glycol having amine at both ends and a molecular weight of 1000

4)    PEG 2000: polyethylene glycol having amine at both ends and a molecular weight of 2000

5)    The copolymer is mixed with a polyamide of 1,3-bisaminomethylcyclohexane and isophthalic acid in a weight ratio of the figure in parentheses

6)    PVP: polyvinyl pyrrolidone

7)    Unit: $ml/hr \cdot mmHg \cdot m^2$

8)    Unit: $\mu m/sec$

9)    Polyamide in polymer alloy (copolymer wt/polyamide wt)

10)    water-soluble polymer

As described on the foregoing pages, the blood compatible materials of the present invention which are made of the polyether-polyamide block copolymer that contains as a constituent the polyamide having a heat of crystallization of no more than 30 mJ/mg have high resistance to thrombus formation. If these materials are worked into dialysis membranes, they exhibit high performance, particularly in the permeation of medium- and high-molecular weight substances; hence, the materials can advantageously be used in hemodialysis. Further, in addition to strong resistance to thrombosis, the polyether-polyamide block copolymer from which the blood compatible materials are made exhibit sufficient mechanical strength to warrant expanded use in various blood-contacting medical devices such as oxigenator, artificial kidneys, blood filters, blood bags, blood circuits, catheters and artificial blood vessels.

Since the polyamide which is a constituent of the block copolymer has a heat of crystallization of no more than 30 mJ/mg, the block copolymer under consideration is soluble in polar organic solvents such as DMSO, DMAc and NMP and, hence, various substrates can be surface treated by coating with this block copolymer. Further, excepting those block copolymers which use wholly aromatic polyamides as a constituent or polymer alloys thereof with polyamides, the block copolymer of the present invention or polymer alloy thereof is melt moldable and, hence, it can be extrusion or injection molded to find use in a broad range of applications.

The blood compatible material of the present invention has an adherent platelet count of 100 or less, preferably 80 or less, more preferably 50 or less in a platelet adhesion test as described later in Examples.

## Claims

1.    A blood compatible material that comprises a polyether-polyamide block copolymer comprising polyether blocks and polyamide blocks having a heat of crystallization of no more than 30 mJ/mg, wherein the polyether-polyamide block copolymer has thermal plasticity.

2. A blood compatible material according to claim 1 wherein recurring units having a cyclic structure account for 100 - 30 mol % of all recurring units in said polyamide blocks.

3. A blood compatible material according to claim 2 wherein at least one of the dicarboxylic acid component and the diamine component making up said recurring units having a cyclic structure, have a cyclic structure.

4. A blood compatible material according to claim 3 wherein said dicarboxylic acid component is isophthalic acid and/or terephthalic acid.

5. A blood compatible material according to claim 3 wherein said diamine component is m-xylylenediamine and/or 1,3-bisaminomethylcyclohexane.

6. A blood compatible material according to claim 1 wherein said polyether blocks are comprised of at least one member selected from the group consisting of polyethylene oxide, polypropylene oxide and polytetramethylene oxide that have a molecular weight of 200 - 20,000.

7. A blood compatible material according to claim 1 wherein the content of said polyether blocks is 2 - 50 wt % of said block copolymer.

8. A blood compatible material according to claim 1 which has an adherent platelet count of 100 or less in a platelet adhesion test.

9. A permselective membrane that comprises the blood compatible material according to claim 1.

10. A blood compatible material that comprises a polymer alloy that comprises

a polyether-polyamide block copolymer which comprises polyether blocks and polyamide blocks having a heat of crystallization of no more than 30 mJ/mg, and,
a polyamide having a heat of crystallization of no more than 30 mJ/mg.

11. A blood compatible material according to claim 10 wherein recurring units having a cyclic structure account for 100 - 30 mol % of all recurring units in said polyamide blocks.

12. A blood compatible material according to claim 11 wherein at least one of the dicarboxylic acid component and the diamine component making up said recurring units having a cyclic structure, have a cyclic structure.

13. A blood compatible material according to claim 12 wherein said dicarboxylic acid component is isophthalic acid and/or terephthalic acid.

14. A blood compatible material according to claim 12 wherein said diamine component is m-xylylenediamine and/or 1,3-bisaminomethylcyclohexane.

15. A blood compatible material according to claim 10 wherein said polyether blocks are comprised of at least one member selected from the group consisting of polyethylene oxide, polypropylene oxide and polytetramethylene oxide that have a molecular weight of 200 - 20,000.

16. A blood compatible material according to claim 10 wherein the content of said polyether blocks is 0.5 - 60 wt % of said polymer alloy.

17. A blood compatible material according to claim 10 which has an adherent platelet count of 100 or less in a platelet adhesion test.

18. A permselective membrane that comprises the blood compatible material according to claim 10.

19. A hollow-fiber membrane that comprises the blood compatible material according to claims 1 or 10.

20. A hollow-fiber membrane according to claim 19 that comprises at least an active layer which is located on inner surface or outer surface of the hollow-fiber, a fingerlike void and porous portions which are located between the active layer and the opposite side of the active layer.

21. A hollow-fiber membrane according to claim 19 that comprises at least an active layer which is located on inner surface or outer surface of the hollow-fiber, and a porous portion that is located inner or outer side to said active layer and which has a microporous structure with continuous open pores.

22. A hollow-fiber membrane according to claim 19 which has an ultrafiltration rate of 2 - 1200 ml/m$^2 \cdot$ hr $\cdot$ mmHg at 37°C.

23. A hollow-fiber membrane according to claim 19 which has a sieving coefficient of no more than 1% for albumin.

24. A process for producing a hollow-fiber membrane of claim 20 that comprises the steps of preparing a spinning solution that consists of 5 - 40 wt % of the block copolymer as recited in claim 1 or the polymer alloy as recited in claim 10 and 95 - 60 wt % of a polar solvent and extruding said spinning solution through the outer tube of a spinneret while, at the same time, extruding a coagulating solution through the inner tube of said spinneret, thereby coagulating said spinning solution.

25. A process according to claim 24 wherein said polar solvent is at least one member of the group consisting of dimethyl sulfoxide, N,N-dimethylformamide, N,N-dimethylacetamide and N-methylpyrrolidone.

26. A process for producing a hollow-fiber membrane of claim 21 that comprises the steps of preparing a spinning solution that consists of 5 - 40 wt % of the block copolymer as recited in claim 1 or the polymer alloy as recited in claim 10, 93 - 40 wt % of a polar solvent and 2 - 20 wt % of a water-soluble polymer and extruding said spinning solution through the outer tube of a spinneret while, at the same time, extruding a coagulating solution through the inner tube of said spinneret, thereby coagulating said spinning solution.

27. A process according to claim 26 wherein said polar solvent is at least one member of the group consisting of dimethyl sulfoxide, N,N-dimethylformamide, N,N-dimethylacetamide and N-methylpyrrolidone and wherein said water-soluble polymer is at least one member of the group consisting of polyethylene glycol, polyvinyl pyrrolidone and methyl cellulose.

**Patentansprüche**

1. Blutverträgliches Material, das ein Polyether-Polyamid-Blockcopolymer umfaßt, das Polyetherblöcke und Polyamidblöcke umfaßt, die eine Kristallisationswärme von nicht mehr als 30 mJ/mg besitzen, wobei das Polyether-Polyamid-Blockcopolymer thermische Plastizität aufweist.

2. Blutverträgliches Material nach Anspruch 1, worin Wiederholungseinheiten mit cyclischer Struktur 100 bis 30 Mol-% aller Wiederholungseinheiten des Polyamidblockes ausmachen.

3. Blutverträgliches Material nach Anspruch 2, worin wenigstens eine der beiden Komponenten, die die Wiederholungseinheiten mit cyclischer Struktur ausmachen, nämlich die Dicarbonsäurekomponente und die Diaminkomponente, eine cyclische Struktur aufweist.

4. Blutverträgliches Material nach Anspruch 3, worin die Dicarbonsäurekomponente Isophthalsäure und/oder Terephthalsäure ist

5. Blutverträgliches Material nach Anspruch 3, worin die Diaminkomponente m-Xylylendiamin und/oder 1,3-Bis(aminomethyl)cyclohexan ist.

6. Blutverträgliches Material nach Anspruch 1, worin die Polyetherblöcke aus wenigstens einem Element bestehen, ausgewählt aus der Gruppe, bestehend aus Polyethylenoxid, Polypropylenoxid und Polytetramethylenoxid, die Molekulargewichte von 200 bis 20000 besitzen.

7. Blutverträgliches Material nach Anspruch 1, worin der Gehalt der Polyetherblöcke 2 bis 50 Gew.-% des Blockcopolymers ausmacht.

8. Blutverträgliches Material nach Anspruch 1, das eine Blutplättchenhaftzahl von 100 oder weniger bei der Blutplättchenhaftprüfung aufweist.

9. Permselektive Membran, die das blutverträgliche Material nach Anspruch 1 umfaßt.

10. Blutverträgliches Material, das eine Polymerlegierung umfaßt, die ein Polyether-Polyamid Blockcopolymer, das Polyether-blöcke und Polyamidblöcke umfaßt, die eine Kristallisationswärme von nicht mehr als 30 mJ/mg besitzen, und ein Polyamid, das eine Kristaliisationswärme von nicht mehr als 30 mJ/mg besitzt, umfaßt.

11. Blutverträgliches Material nach Anspruch 10, worin Wiederholungseinheiten mit cyclischer Struktur 100 bis 30 Mol-% aller Wiederholungseinheiten des Polyamidblockes ausmachen.

12. Blutverträgliches Material nach Anspruch 11, worin wenigstens eine der beiden Komponenten, die die Wiederholungseinheiten mit cyclischer Struktur ausmachen, nämlich die Dicarbonsäurekomponente und die Diaminkomponente, eine cyclische Struktur aufweist.

13. Blutverträgliches Material nach Anspruch 12, worin die Dicarbonsäurekomponente Isophthalsäure und/oder Terephthalsäure ist.

14. Blutverträgliches Material nach Anspruch 12, worin die Diaminkomponente M-Xylylendiamin und/oder 1,3-Bis(aminomethyl)cyclohexan ist.

15. Blutverträgliches Material nach Anspruch 10, worin die Polyetherblöcke aus wenigstens einem Element bestehen, ausgewählt aus der Gruppe, bestehend aus Polyethylenoxid, Polypropylenoxid und Polytetramethylenoxid, die Molekulargewichte von 200 bis 20000 besitzen.

16. Blutverträgliches Material nach Anspruch 10, worin der Gehalt der Polyetherblöcke 0,5 bis 60 Gew.-% der Polymerlegierung ausmacht.

17. Blutverträgliches Material nach Anspruch 10, das eine Blutplättchenhaftzahl von 100 oder weniger bei der Blutplättchenhaftprüfung aufweist.

18. Permselektive Membran, die das blutverträgliche Material nach Anspruch 10 umfaßt.

19. Hohlfasermembran, die das blutverträgliche Material nach Anspruch 1 oder Anspruch 10 umfaßt.

20. Hohlfasermembran nach Anspruch 19, die wenigstens eine aktive Schicht, die auf der inneren Oberfläche oder auf der äußeren Oberfläche der Hohlfaser angeordnet ist, eine fingerartige Höhlung und poröse Bereiche, die zwischen der aktiven Schicht und der gegenüberliegenden Seite der aktiven Schicht angeordnet sind, umfaßt.

21. Hohlfasermembran nach Anspruch 19, die wenigstens eine aktive Schicht, die auf der inneren Oberfläche oder auf der äußeren Oberfläche der Hohlfaser angeordnet ist, und einen porösen Bereich, der im Bezug auf die aktive Schicht zur inneren Seite oder zur äußeren Seite hin angeordnet ist und eine Mikroporenstruktur mit durchgehenden, offenen Poren aufweist, umfaßt.

22. Hohlfasermembran nach Anspruch 19, die eine Ultrafiltrationsgeschwindigkeit von 2 bis 1200 ml/m$^2 \cdot$ h $\cdot$ mmHg bei 37°C besitzt.

23. Hohlfasermembran nach Anspruch 19, die für Albumin einen Siebkoeffizienten von nicht mehr als 1% aufweist.

24. Verfahren zur Herstellung einer Hohlfasermembran nach Anspruch 20, das folgende Schritte umfaßt:

   • Herstellen einer Spinnlösung, die aus 5 bis 40 Gew.-% des Blockcopolymers nach Anspruch 1 oder der Polymerlegierung nach Anspruch 10 und 95 bis 60 Gew.-% eines polaren Lösungsmittels besteht, und
   • Extrudieren dieser Spinnlösung durch die äußere Röhre einer Spinndüse, während gleichzeitig eine Koagulierlösung durch die innere Röhre der Spinndüse extrudiert wird, wodurch die Spinnlösung koaguliert.

25. Verfahren nach Anspruch 24, worin das polare Lösungsmittel wenigstens ein Element der Gruppe ist, bestehend aus Dimethylsulfoxid, N,N-Dimethylformamid, N,N-Dimethylacetamid und N-Methylpyrrolidon.

26. Verfahren zur Herstellung einer Hohlfasermembran nach Anspruch 21, das folgende Schritte umfaßt:

   • Herstellen einer Spinnlösung, die aus 5 bis 40 Gew.-% des Blockcopolymers nach Anspruch 1 oder der Polymerlegierung nach Anspruch 10, 93 bis 40 Gew.-% eines polaren Lösungsmittels und 2 bis 20 Gew.-% eines wasserlöslichen Polymers besteht, und

- Extrudieren der Spinnlösung durch die äußere Röhre einer Spinndüse, während gleichzeitig eine Koagulierlösung durch die innere Röhre der Spinndüse extrudiert wird, wodurch die Spinnlösung koaguliert.

27. Verfahren nach Anspruch 26, worin das polare Lösungsmittel wenigstens ein Element der Gruppe ist, bestehend aus Dimethylsulfoxid, N,N-Dimethylformamid, N,N-Dimethylacetamid und N-Methylpyrrolidon, und worin das wasserlösliche Polymer wenigstens ein Element der Gruppe ist, bestehend aus Polyethylenglycol, Polyvinylpyrrolidon und Methylcellulose.

**Revendications**

1. Matière hémocompatible, comprenant un copolymère séquencé polyéther-polyamide comprenant des blocs polyéther et des blocs polyamide ayant une chaleur de cristallisation inférieure ou égale à 30 mJ/mg, dans laquelle le copolymère séquencé polyéther-polyamide fait preuve de plasticité thermique.

2. Matière hémocompatible selon la revendication 1, dans laquelle les motifs récurrents ayant une structure cyclique représentent 100 à 30 % en moles de tous les motifs récurrents dans lesdits blocs polyamide.

3. Matière hémocompatible selon la revendication 2, dans laquelle au moins un des composants acide dicarboxylique et diamine constituant lesdits motifs récurrents ayant une structure cyclique a une structure cyclique.

4. Matière hémocompatible selon la revendication 3, dans laquelle ledit composant acide dicarboxylique est de l'acide isophtalique et/ou de l'acide téréphtalique.

5. Matière hémocompatible selon la revendication 3, dans laquelle ledit composant diamine est dc la m-xylylènediamine et/ou du 1,3-bisaminométhylcyclohexane.

6. Matière hémocompatible selon la revendication 1, dans laquelle lesdits blocs polyéther comprennent au moins un élément sélectionné dans le groupe constitué par le poly(oxyde d'éthylène), le poly(oxyde de propylène) et le poly(oxyde de tétraméthylène) ayant une masse moléculaire de 200 à 20 000.

7. Matière hémocompatible selon la revendication 1, dans laquelle la proportion desdits blocs polyéther est de 2 à 50 % en poids dudit copolymère séquencé.

8. Matière hémocompatible selon la revendication 1, laquelle présente une numération de plaquettes adhérentes inférieure ou égale à 100 au cours d'un essai d'adhésivité des plaquettes.

9. Membrane à perméabilité sélective, qui comprend la matière hémocompatible selon la revendication 1.

10. Matière hémocompatible qui comprend un alliage polymère comprenant

   un copolymère séquencé polyéther-polyamide qui comprend des blocs polyéther et des blocs polyamide ayant une chaleur de cristallisation inférieure ou égale à 30 mJ/mg et
   un polyamide ayant une chaleur de cristallisation inférieure ou égale à 30 mJ/mg.

11. Matière hémocompatible selon la revendication 10, dans laquelle les motifs récurrents ayant une structure cyclique représentent 100 à 30 % en moles de tous les motifs récurrents dans lesdits blocs polyamide.

12. Matière hémocompatible selon la revendication 11, dans laquelle au moins un des composants acide dicarboxylique et diamine constituant lesdits motifs récurrents ayant une structure cyclique a une structure cyclique.

13. Matière hémocompatible selon la revendication 12, dans laquelle ledit composant acide dicarboxylique est de l'acide isophtalique et/ou de l'acide téréphtalique.

14. Matière hémocompatible selon la revendication 12, dans laquelle ledit composant diamine est de la m-xylylènediamine et/ou du 1,3-bisaminométhylcyclohexane.

15. Matière hémocompatible selon la revendication 10, dans laquelle lesdits blocs polyéther comportent au moins un élément sélectionné dans le groupe constitué par le poly(oxyde d'éthylène), le poly(oxyde de propylène) et le poly(oxyde de tétraméthylène) ayant une masse moléculaire de 200 à 20 000.

16. Matière hémocompatible selon la revendication 10, dans laquelle la proportion desdits blocs polyéther est de 0,5 à 60 % en poids dudit alliage polymère.

17. Matière hémocompatible selon la revendication 10, qui présente une numération de plaquettes adhérentes inférieure ou égale à 100 dans un test d'adhésivité des plaquettes.

18. Membrane à perméabilité sélective qui comprend la matière hémocompatible selon la revendication 10.

19. Membrane en fibre creuse qui comprend la matière hémocompatible selon la revendication 1 ou 10.

20. Membrane en fibre creuse selon la revendication 19, qui comprend au moins une couche active située sur la surface interne ou la surface externe de la fibre creuse, un vide en forme de doigt et des parties poreuses situées entre la couche active et la face opposée de la couche active.

21. Membrane en fibre creuse selon la revendication 19, qui comprend au moins une couche active située sur la surface interne ou la surface externe de la fibre creuse et une partie poreuse située intérieurement ou extérieurement par rapport à ladite couche active et qui a une structure microporeuse présentant des pores ouverts continus.

22. Membrane en fibre creuse selon la revendication 19, qui présente un débit d'ultrafiltration de 2 à 1 200 $ml/m^2 \cdot h \cdot mmHg$ à 37°C.

23. Membrane en fibre creuse selon la revendication 19, qui présente un coefficient de tamisage inférieur ou égal à 1 % pour l'albumine.

24. Procédé de production d'une membrane en fibre creuse selon la revendication 20, lequel comprend les étapes consistant à préparer une solution à filer constituée de 5 à 40 % en poids du copolymère séquencé selon la revendication 1 ou de l'alliage polymère selon la revendication 10 et de 95 à 60 % en poids d'un solvant polaire et à extruder ladite solution à filer par le tube externe d'une filière tout en extrudant simultanément une solution coagulante par le tube interne de ladite filière, en coagulant ainsi ladite solution à filer.

25. Procédé selon la revendication 24, dans lequel ledit solvant polaire est au moins un élément du groupe constitué par le diméthylsulfoxyde, le N,N-diméthylformamide, le N,N-diméthylacétamide et la N-méthylpyrrolidone.

26. Procédé de production d'une membrane en fibre creuse selon la revendication 21, lequel comprend les étapes consistant à préparer une solution à filer constituée de 5 à 40 % en poids du copolymère séquencé selon la revendication 1 ou de l'alliage polymère selon la revendication 10, de 93 à 40 % en poids d'un solvant polaire et de 2 à 20 % en poids d'un polymère hydrosoluble et à extruder ladite solution à filer par le tube externe d'une filière tout en extrudant simultanément une solution coagulante par le tube interne de ladite filière, en coagulant ainsi ladite solution à filer.

27. Procédé selon la revendication 26, dans lequel ledit solvant polaire est au moins un élément du groupe constitué par le diméthylsulfoxyde, le N,N-diméthylformamide, le N,N-diméthylacétamide et la N-méthylpyrrolidone et dans lequel ledit polymère hydrosoluble est au moins un élément du groupe constitué par le polyéthylèneglycol, la polyvinylpyrrolidone et la méthylcellulose.

# FIG. 1

SC curve for aqueous dextran solutions
as treated by the hollow-fiber
membrane of Example 42

# FIG. 2

SC curve for aqueous dextran solutions
as treated by the hollow-fiber
membrane of Example 52

## FIG. 3

SEM of cross section of the hollow-fiber
membrane of Example 42

## FIG. 4

SEM of cross section of the hollow-fiber
membrane of Example 52

FIG. 5

# FIG.6

EP 0 600 793 B1

FIG.7